# EUROPEAN PATENT SPECIFICATION

(11) **EP 1 599 556 B1**
(45) Date of publication and mention of the grant of the patent: **25.06.2008**
(21) Application number: 04712572.9
(22) Date of filing: 19.02.2004
(51) Int. Cl.: C09J 7/02, C09J 153/02

(54) **LABEL STOCK WITH IMPROVED DIE-CUTTING PERFORMANCE**
ETIKETTENVORMATERIAL MIT VERBESSERTEM STANZVERHALTEN
FILM A ETIQUETTE PRESENTANT UNE PERFORMANCE AMELIOREE DE DECOUPAGE A L'EMPORTE-PIECE

(30) Priority: 21.02.2003 EP 03100428; 12.06.2003 EP 03101715; 30.06.2003 EP 03077043
(43) Date of publication of application: 30.11.2005
(60) Divisional application: 07075651.5
(73) Proprietor: KRATON Polymers Research B.V., 1030 BH Amsterdam (NL)
(72) Inventor: DUPONT, Martine J., Kraton Polymers Research S.A., B-1348 Ottignies - Louvain-La-Neuve (BE); MAYENEZ, Catherine, Kraton Polymers Research S.A., B-1348 Ottignies - Louvain-La-Neuve (BE)
(74) Representative: Kortekaas, Marcel C.J.A.
(86) International application number: PCT/EP2004/050169
(87) International publication number: WO 2004/074392

(56) References cited:
- EP-A- 0 027 606
- WO-A-02/057386
- DE-A- 2 942 128
- US-A- 5 182 323
- US-A- 5 589 542
- PATENT ABSTRACTS OF JAPAN vol. 1995, no. 06, 31 July 1995 (1995-07-31) & JP 7 076673 A (OJI KAKO KK), 20 March 1995 (1995-03-20)
- PATENT ABSTRACTS OF JAPAN vol. 1995, no. 03, 28 April 1995 (1995-04-28) & JP 6 340859 A (DAICEL CHEM IND LTD), 13 December 1994 (1994-12-13)
- PATENT ABSTRACTS OF JAPAN vol. 0180, no. 40 (C-1155), 21 January 1994 (1994-01-21) & JP 5 263056 A (ASAHI CHEM IND CO LTD), 12 October 1993 (1993-10-12)

## Description

### Background art

Pressure sensitive adhesive ("PSA") labels are highly desirable for a wide variety of purposes. For instance, pages of peelable label stock are available which can be fit into standard printers to print address labels, etc. The preaddressed labels are then peeled off and put on envelopes. There are a wide variety of uses for such labels and many varieties, sizes, and types are available commercially. Alternatively labels may also be used in the packaging industry ("permanent labels").

One method for producing sheets, etc. of PSA labels involves providing a base sheet (or roll) which is generally coated with a release coating. Release coatings are materials from which the label can be peeled away such that the adhesive is not removed from the label and retaining its adhesive properties. Thus, the label then has the capability of being adhered to another substrate.

A separate sheet of label material, so-called face stock, which can be printed or written upon, is formed and a label adhesive is coated on the side of the sheet opposite to the side upon which it is intended to be printed or written upon. The label sheet is then adhered to the release coating sheet by placing the adhesive surface in contact with the release coating surface. For paper labels, the adhesive is usually coated on the release material and this construction is then laminated with the paper. This is called "transfer coating". The printing step happens afterwards. For filmic labels, the adhesive might be coated on the filmic substrate first (depending on the nature of the substrate).

During the label manufacture, a laminate of face stock (either paper or film, either coloured or transparent), PSA layer and release liner is passed through an apparatus that converts the laminate to yield commercially useful labels and label stock. The process involved in the converting operation includes printing, die cutting and matrix stripping to leave labels on a release liner. The cost of converting a laminate into a finished product is a function of the speed at which the various processing operations occur. While the nature of all layers of the laminate can impact cost of convertibility, the adhesive layer is an important limiting factor in ease and cost of convertibility. This is in consequence of its viscoelastic nature, which hampers precise and clean penetration of a die in die-cutting operations and promotes adherence to cutting blades. Stringiness of the adhesive also impacts matrix-stripping operations, which follow die-cutting operations.

Emulsion acrylic adhesives convert very well and this makes them very successful in the label industry. However they exhibit poorer adhesive performance compared to styrenic block copolymers-based adhesives, in particular SIS based adhesives (for example: water sensitivity, lower aggressive tack or lower adhesion on polyolefins), wherein SIS stands for poly(styrene-isoprene-styrene) block copolymer. Moreover, acrylic polymer emulsions require handling large volumes of liquid and subsequent liquid removal. They also perform poorer at low temperatures.

Styrenic block copolymers, of the formula A-B-A (wherein A stands for polystyrene blocks and B stands for elastomeric blocks typically composed of (co)polymerized conjugated dienes) and variations thereof, are used in a variety of PSA applications because of their formulation flexibility, their excellent adhesive properties on a wide range of substrates and their hot-melt processability. They are particularly suitable for tape applications due to their excellent cohesion. However, in label applications the styrenic block copolymers have not yet established a major market position.

The inherently good elastic behaviour of a high performance SIS block copolymer turns out to be an obstacle for its use in labels because it reduces the die-cuttability of the finished label. Thus, when converting the laminate into labels by the process mentioned above, there is label entrainment in the waste matrix, tension is put on the waste matrix which is then distorted, causing problems down line the die cutting step.

It has been found before that this limitation of styrenic block copolymers can be overcome by using grades that comprise a high diblock content, e.g., of general formula A-B. For instance, in the leaflets "Communications from KPRL", issues 7-2000 and 8-2000, released by KRATON Polymers in 2000, the test results of various SIS grades with different diblock content have been presented.

Although PSAs based on KRATON^{®} D-1163NS or KRATON^{®} D-1113 (SIS with diblock content of 40%, respectively 55%) have been found perfectly suited for label applications, further improvement in respect of die-cutting behavior remains desirable. It is an object of the present invention to provide label stock with improved die cutting behavior, as well as the labels produced there from and the adhesive layer used in its preparation.

On the other hand, from WO 02/057386 pressure sensitive adhesive compositions were known, comprising:
1. a styrenic block copolymer of the general formula A-C-A, wherein A is a poly(vinyl aromatic compound) block and C is a mixed copolymerized polymer block of butadiene and isoprene in a weight ratio of 30:70 to 70:30,
2. 50 to 400 parts by weight of a tackifying resin, and preferably from 100 to 300 parts by weight per 100 parts by weight of block copolymer component (a).

Although said pressure sensitive adhesive compositions were mentioned to be used in a label stock comprising a laminate of a face stock, an adhesive layer comprising said adhesive composition and a release liner, only adhesive compositions were actually disclosed, comprising a block copolymer (a), having a diblock copolymer content of at most 19 % i.e. a coupling efficiency in the range of from 81 to 87 % in Table 1, page 19. Moreover, no disclosed adhesive compositions were actually applied for label stock preparation.

### Disclosure of the Invention

Accordingly, the present invention relates to label stock comprising a laminate of face stock, a PSA layer and a release liner, wherein the PSA layer comprises
(a) at least one block copolymer, comprising at least two terminal blocks of poly(vinyl aromatic compound) and at least one midblock of a randomly copolymerized mixture of isoprene (I) and butadiene (B) used in a weight ratio of (I):(B) of 35:65 to 80:20, and having a poly(vinyl aromatic compound) content in the range of from 10 to 35 wt% and an apparent molecular weight in the range from 180,000 to 400,000 g/mole;
(b) at least one diblock copolymer, comprising one block of poly(vinyl aromatic compound) and one block of polymerized butadiene, isoprene or mixtures thereof;
(c) a tackifying resin; and
(d) optionally a plasticizer
wherein component (a) is a thermoplastic elastomer, component (b) comprises from 30 to 85% by weight of the mixture of components (a) and (b), and wherein on 100 parts by weight of the mixture of components (a) and (b), from 20 to 450 parts by weight component (c) is used.

### Brief Description of Drawings

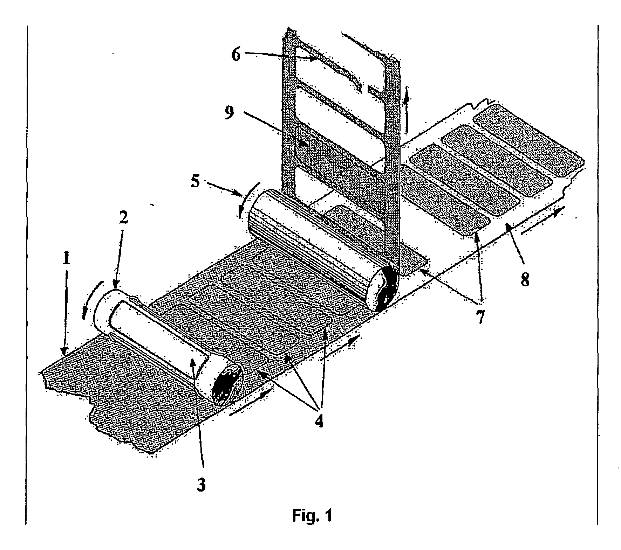

The basic method for producing labels from roll label stock by label die cutting is shown in Figure 1. The roll label stock 1 is fed through a rotary die 2 which has several cutters 3 which produce the desired shape of the cut out 4 in the roll label stock 1. The roll label stock 1 then continues on to the stripping roller 5 which removes the matrix flagging 6 from the roll stock 1, leaving behind the die cut labels 7.

With respect to this process it will be understood that it is important that the rotary die 2 be able to cut through the adhesive and the top layer of roll label stock 1. The adhesive must be strong enough, however, to leave the label behind on the release liner 8 of roll label stock 1.

It should also be realized that the die-cutting behaviour is influenced by the blade angle of rotary die 2 and the speed of the process. The narrower the angle, the sharper but also the more expensive and fragile the blades. For this reason, it is preferred to use as large an angle as is possible. Ideally high speeds, even at high blade cutting angles (e.g., more than 50 m/min at a blade cutting angle of 110°) should be achieved.

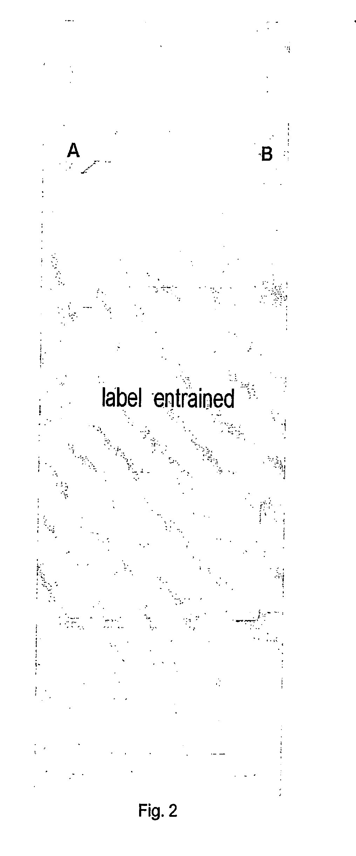

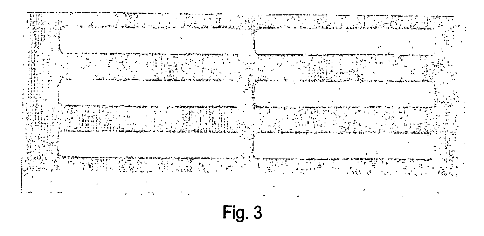

Figures 2 and 3 represent two different label designs for testing the die-cutting ease. Figure 2 represents a design where the matrix is very thin between points A and B. This makes this design very sensitive to matrix break between these points. The die-cutting ease is then expressed as the number of defects (label entrainments or matrix breaks) occuring during the die-cutting test (method 1).

Figure 3 represents also a fairly difficult label design on a die-cutting ease point of view; in this case, the die-cutting ease is expressed as the maximum speed the die-cutting operation can be performed without problem (method 2).

### Mode(s) for carrying out the invention

### Component (a)

The main block copolymer component used in the adhesive composition is a block copolymer, having a structure represented by the general formulae

S-(I/B)-S (1)

or

[S-(I/B)]ₙX (2),

optionally mixed with minor amounts of one or more block copolymers selected from the group S-B-S, S-I-S, and radial variations thereof wherein S represents a poly(vinyl aromatic compound) block, (I/B) represents a block of a randomly copolymerized mixture of isoprene and butadiene, wherein the weight ratio between isoprene and butadiene is in the range of from 35:65 to 80:20, preferably from 40:60 to 80:20 and more preferably from 70:30 to 80:20, wherein n is an integer equal to or greater than 2, and wherein X is the residue of a coupling agent. Such block copolymers are known to behave as thermoplastic elastomers, given the proper choice of molecular weight, and total content of vinyl aromatic compound.

As an example of the aromatic vinyl compound useful in the practice of the present invention, may be mentioned styrene, alpha-methylstyrene, p-methylstyrene, o-methylstyrene, p-tert.butylstyrene, dimethylstyrene, and vinyl naphthalene or mixtures thereof. Of these, styrene is particularly preferred from the viewpoints of easy availability, reactivity, physical properties of the resulting block copolymers. The A polymer block may contain minor amounts of comonomers other than an aromatic vinyl compound, e.g., up to 5 wt% of a copolymerizable monomer such as butadiene and/or isoprene (based on the weight of the total block). Most preferred are A blocks derived from substantially pure styrene. These polymer blocks A preferably have a true molecular weight in the range from 9,500 to 25,000.

The mixed polymer midblock (I/B) is made of butadiene and isoprene as copolymerizing monomers, although it too may contain minor amounts of other comonomers, e.g. up to 5 wt% of a copolymerizable monomer such as styrene (based on the weight of the total block), but mixtures of substantially pure isoprene and butadiene are preferred.

In the block copolymers (a) according to the present invention, the proportion of bound aromatic vinyl compound is in the range of 10-35 wt%, preferably 13-30 wt% based on the total block copolymer.

Each block copolymer (a) to be applied in the adhesive compositions according to the present invention preferably has an apparent molecular weight (Mw, expressed in terms of polystyrene) ranging from 180,000 to 400,000 g/mol and more preferably from 200,000 to 320,000 g/mol, as determined by gel permeation chromatography (GPC, analogous to the method described in ASTM D5296-97).

The block copolymers to be applied in the adhesive compositions according to the present invention each preferably contain 1,2-vinyl bonds and/or 3,4-vinyl bonds in a proportion in the range of from 5 to 40 wt% based on the weight of the conjugated diene, and preferably from 5 to 20 wt% based on the weight of conjugated diene.

The block copolymers according to the present invention each have only one peak on loss tangent (tan δ) attributable to the mixed butadiene/isoprene polymer block at a temperature of -50°C or below.

Said block copolymers to be applied as main component (a) in the adhesive composition, have a randomly copolymerized block (I/B), which means that the mixed midblock shows no significant single homopolymer block formation. They can be prepared as described in WO 02057386 (KRATON) 25.07.2002, incorporated herein by reference.

The block copolymers according to the present invention can be made e.g. by coupling living diblock copolymer prepared by anionic polymerization with a coupling agent.

As examples of the coupling agent, may be mentioned tin coupling agents such as tin dichloride, monomethyltin dichloride, dimethyltin dichloride, monoethyltin dichloride, diethyltin dichloride, methyltin trichloride, monobutyltin dichloride, dibutyltin dibromide, monohexyltin dichloride and tin tetrachloride; halogenated silicon coupling agents such as dichlorosilane, monomethyldichlorosilane, dimethyldichlorosilane, monoethyldichlorosilane, diethyldichlorosilane, monobutyldichlorosilane, dibutyldichlorosilane, monohexyldichlorosilane, dihexyldichlorosilane, dibromosilane, monomethyldibromosilane, dimethyldibromosilane, silicon tetrachloride and silicon tetrabromide; alkoxysilanes such as tetramethoxysilane; divinyl aromatic compounds such as divinylbenzene and divinylnaphthalene; halogenated alkanes such as dichloroethane, dibromoethane, methylene chloride, dibromomethane, dichloropropane, dibromopropane, chloroform, trichloroethane, trichloropropane and tribromopropane; halogenated aromatic compounds such as dibromobenzene; epoxy compounds such as the diglycidyl ether of bisphenol-A and the like (e.g., EPON^{™} 825 or EPON^{™} 826 diglycidyl ether) and other coupling agents such as benzoic esters, CO, 2- and 1-chloro-1,3-butadiene. Of these, EPON^{™} 826 diglycidyl ether, dibromobenzene, tetramethoxysilane or other tetra(alkoxy)silanes are preferred.

The block copolymer components (a) may also be prepared by a full sequential polymerization process (resulting in a product of general formula (1), with re-introduction of initiator before or during the step 2 polymerization (preparation of the I/B block), resulting in the in-situ formation of a diblock copolymer (component (b)).

### Component (b)

Component (b) may be the finished, uncoupled diblock copolymer left as a component in component (a) which is synthesized by coupling a living diblock copolymer. In this preferred embodiment, the polymer blocks of both components will be identical. It may also be the in-situ produced diblock copolymer as the result of re-introduction of the initiator. Alternatively, a different diblock copolymer may be used, including poly(styrene-butadiene) and poly(styrene-isoprene) diblock copolymers.

It will be appreciated that component (b) may comprise one diblock copolymer S-(I/B) or a mixture of diblock copolymers S-(I/B) and S-B or S-(I/B) and S-I.

The component (b) preferably occurs in a proportion of from 35 to 85 wt%, relative to the weight of components (a) and (b).

The (I):(B) weight ratio in component (b) can vary in the range of from 10:90 to 80:20, but preferably in the range of from 35:65 to 80:20.

According to a more preferred embodiment of the present invention, component (a) will consist of a triblock copolymer of formula 1, while component (b) is formed by the terminated intermediate diblock copolymer S-(I/B), from which the component (a) block copolymer has been derived by coupling.

According to a most preferred embodiment of the present invention, said triblock copolymer S-(I/B)-S and S-(I/B) both have a (I):(B) ratio in the range of from 70:30 to 80:20.

Indicated (I/B) weight ratios for the diblock copolymer component (b) throughout the specification and claims are, in the event that component (b) is a mixture, the average weight ratios relative to the weight of the total mixture.

### Components (c) and (d)

Tackifying resins and plasticizers, as well as other components like antioxidants and the like, for use in PSA compositions based on styrenic block copolymers are known. Such PSAs are, for instance, described in Chapter 13 of the Handbook of Pressure Sensitive Adhesive Technology, 2nd ed. by Donatas Satas (ISBN 0-442-28026-2). On pages 326-328 various rubber phase associated resins are disclosed, e.g., aliphatic olefin-derived resins, rosin esters, polyterpenes and terpene phenolic resins derived from petroleum or terpentine sources, which provide tack to the styrenic block copolymer. In Table 13-10 of this handbook label formulations based on 100 parts of KRATON® D-1112X and D-1117X are described, having good die cuttability, further comprising 140 parts of hydrocarbon tackifying resin, such as WINGTACK^{™} 95 (Goodyear) or ESCOREZ^{™} 1310 (Exxon), 40 parts of plasticizer such as SHELLFLEX^{™} 371 (Shell) and 2 parts of phenolic antioxidant, such as IRGANOX^{™} 1010 or IRGANOX^{™} 565 (Ciba-Geigy) or antioxidant 330 (Ethyl Corp.) A long list of suitable tackifying resins may be found in European patent application EP0802251A, herewith incorporated by reference. Particularly suitable tackifying resins are those having an average aromaticity (in relative percentage of aromatic protons as determined by proton-NMR) in the range of from 3 to 14% and preferably from 4 to 12%. Preferred types of resins are the mixed aliphatic/aromatic resins, mentioned in WO 02057386. An example of a suitable tackifying resin is WINGTACK^{™} 86, a modified aliphatic hydrocarbon resin with a softening point of 86°C, and an aromaticity of 9.6.

The optional fourth component (d) of the adhesive formulation of the present invention is oil. It preferably is present in an amount from 10 to 60 parts by weight per 100 parts of the mixture of components (a) and (b). Rubber compounding oils are well known in the art and include both high saturates content oils and naphthenic oils. Preferred plasticizer oils are highly saturated oils, e.g., TUFFLO^{™} 6056 and 6204 oil made by Arco and naphthenic process oils, e.g., SHELLFLEX^{™} 371 and 6371 or EDELEX 956 oil made by Shell Chemical Company. The preferred amount of oil is 20 to 50 parts by weight so as to obtain the appropriate glass transition temperature and tack (label formulations typically contain about 40 phr of oil).

These adhesive formulations can be made by the typical hot melt mixing process, e.g. using a sigma blade mixer. The compositions of the present invention may be modified further with the addition of other antioxidants and stabilizers without departing from the scope of this invention.

The process of preparing a laminate of a face stock, PSA layer and a release liner is also known. Thus, the PSA layer may be applied without using any solvent (e.g., hot-melt) or in the form of its solution to a base material such as paper or a plastic film by means of a proper coater.

### EXAMPLES

A number of different block copolymers and tackifying resins were used in combination with oil as plasticizer and antioxidant to make adhesive formulations for testing herein. The materials which were used in these experiments are listed in Table 1. PICCOTAC^{™} 1094-E resin is an aliphatic hydrocarbon resin and WINGTACK^{™} 86 resin has been defined herein before. The resins were used in an amount of 150 parts by weight on 100 parts by weight of the mixture of components (a) and (b) in both compositions. EDELEX^{™} 956, a naphthenic mineral oil, was used as plasticizer in both compositions, in an amount of 40 parts by weight. Finally, the formulation comprised 3 parts by weight of IRGANOX^{™} 1010 as antioxidant.

The tensile, adhesive, and process properties of these formulations were measured.
- 180° peel adhesion (PA) was determined by Pressure Sensitive Tape Council Method No.1. Large numbers indicate high strength when peeling a test tape from a steel substrate. The abbreviation "cf" means cohesive failure: this is the case when the failure occurs inside the adhesive and not at the interface with the substrate.
- Loop tack (LT) was determined using a (TLMI) loop tack tester. High numbers for LT indicate aggressive tack.
- Rolling Ball Tack (RBT) is the distance a steel ball rolls on the adhesive film with a standard initial velocity (Pressure Sensitive Tape Council Test No. 6). Small numbers indicate aggressive tack.
- Holding Power (HP) is the time required to pull a standard area (2.54 cm x 2.54 cm) of tape from a standard test surface (steel, Kraft paper) under a standard load (500g), in shear at 2° antipeel (FTM (Finat Test Method) 8). Long times indicate high adhesive strength. The test was here conducted at 70°C.
- Hot Melt Viscosity (HMV) : the melt viscosity of an adhesive is measured with a Brookfield Digital Viscometer. The HMV are reported in Pa.s together with the temperature. Here it was measured at 160°C, according to ASTM D-3236-73.
- Pourability is a test wherein the adhesive hot-melt is put in an oven at 160°C for 24 hours and its pourability is then qualitatively assessed: 1: flows very well / 2: flows well but slowly / 3: flows with difficulty and highly viscous / 4: does not flow.
- Die-cutting behaviour:
   Test 1: A specific label design as shown in Figure 2 was used to assess the die-cutting ease. In this specific design of Figure 2, the area between points A and B is prone to show matrix breakage. The die-cutting difficulty was expressed as the amount of defects detected on an area of 2 m². Two types of defects were distinguished: matrix break (MB), leaving a part of the matrix on the top of the label, and label entrainment (LE). The speeds ranged from 10 to 80 m/min.
   Test 2: Here another label design (Figure 3) was chosen, wherein again the results are expressed as the speed achievable without problem during the die-cutting operation. The maximum speed limit of the equipment used for this test (Focus^{™} label converting equipment) is 80 m/min.

The results are recorded in Tables 2 and 3. As shown in Table 2, both adhesives provide labels having acceptable properties. As shown in Table 3, the label stock based on polymer composition A has the best die-cutting behaviour.

**Table 1**

| Polymer | Type | PSC | DBC | (I/B) | MM |
|---|---|---|---|---|---|
| | | (%) | (%wt) | ratio | (kg/mol) |
| KRATON® D-1113 | S-I-S + S-I | 16 | 55 | 100/0 | 245 |
| PC. A | S-I/B-S + S-I/B | 16 | 55 | 40/60 | 221 |

The abbreviation "PC" stands for Polymer Composition; PSC is polystyrene content; DBC is the amount of diblock (b) in the polymer composition, MM is the apparent molecular mass, expressed in polystyrene equivalent, as measured by GPC.

**Table 2**

| Formulation base on ... | Comparative PC. 1: KRATON KD1113 + PICCOTAC 1094E + EDELEX 956 | PC. A + WINGTACK 86 + EDELEX 956 |
|---|---|---|
| Mixing T, °C | 160 | 160 |
| PA, N/25 mm | paper tear | paper tear |
| LT, N/25 mm | 27 | 24 |
| RBT, cm | 8 | 6 |
| HP at 70°C, 500g, h | 2.3 | 1.4 |

Polymer composition A has a slightly better RBT performance, and a slightly worse LT and HP performance than comparative composition 1. On balance, these two compositions perform equally well.

**Table 3 (Die-cutting ease - test 2)**

| Formulation based on ... | KD1113 | PC. A |
|---|---|---|
| Die cutting behaviour, depending on blade angle | | |
| 60° | < 50 m/min | >80 m/min |
| 75° | < 10 m/min | >80 m/min |
| 110° | < 6 m/min | >80 m/min |

The die cutting behaviour has been tested by Test method 2. The die cutting behaviour of Polymer composition A (80 is the speed limit of the converting equipment) greatly outperforms that of the composition based on KD1113, which illustrates the superior properties of Polymer composition A.

Die-cutting experiments were also performed using the hereinbefore specified polymers and compositions:

**Table 4 (Formulation tested:)**

| Composition | amount |
|---|---|
| Mixture of Polymer (a) and (b) | 100 pbw |
| WINGTACK 86 | 150 pbw |
| EDELEX 956 | 40 pbw |
| IRGANOX 1010 | 3 pbw |

The abbreviation pbw stands for parts by weight.

**Table 5 (Polymers tested:)**

| Polymer | Type | I/B ratio | PSC (%) | DBC (%) | MM (Kg/mol) |
|---|---|---|---|---|---|
| PC. B | S-I/B-S + | 76/24 (a) | 17.5 | 79 | 245 |
| | S-I/B | 76/24 (b) | | | |
| PC. C | (S-I/B)₃ + | 70/30 (a) | 19 | 37 | 292 |
| | S-I/B | 70/30 (b) | | | |
| Comp. PC. 2 | S-I/B-S + | 27/73 (a) | 17 | 71 | 260 |
| | S-I/B | 27/73 (b) | | | |
| PC. E | (S-I/B)₃ + | 39/61 (a) | 17.5 | 65 | 354 |
| | S-I/B | 39/61 (b) | | | |
| EUROPRENE^{™} 1205 | S-B | 0/100 (b) | 25 | 100 | 120 |
| PC. F | S-I/B-S + | 41/59 (a) | 21 | 80 | |
| | S-I/B + S-B | 13/87 (b) | | | |
| Comp. PC. 3 | S-I-S + | 100/0 (a) | 21 | 80 | |
| | S-I + S-B | 33/67 (b) | | | |
| Comp. PC. 4 | S-I/B-S | 51/49 (a) | 17 | 0 | 267 |

The polymer composition F contains 44% of the polymer composition A and 56% EUROPRENE 1205. The comparative polymer composition 3 contains 44% of the KD1113 and 56% EUROPRENE 1205. For all other polymer compositions (A->E, KD1113, EUROPRENE 1205 and comparative example), the I/B ratio is similar in (a) and (b). The I/B ratio in (b) may come from two diblocks different in nature or from a diblock with mixed midblock.

**Table 6**

| Formulation based on... | PC. B | PC. C | Comp. PC. 2 | PC. E | PC. F | Comp. PC. 3 | Comp. PC. 4 |
|---|---|---|---|---|---|---|---|
| HMV, Pa.s | 16 | 31,1 | 30,2 | 47 | 27,7 | 20,7 | 225 |
| Pourability | 1 | 1 | 1 | 1.5 | 1.5 | 3 | 4 |
| PA, N/25 mm | 27cf | 15 | 28cf | 16 | 15cf | 17cf | 14 |
| LT, N/25 mm | 20 | 16 | 19 | 16 | 14 | 11 | 18 |
| RBT, cm | 4 | - | - | 1 | 1 | 1 | 3 |

The adhesive properties are in all cases well suited for PSA label application. The compositions with the highest diblock content show cohesive failure at the PA test. It has to be noted that formulation with similar amount of diblock based on blends SIS/SB (comparative polymer comp. 3) shows lower stability (pourability after 24 hours starts to become critical) compared to SIBS/SB. This is a considerable advantage hot-melt processing of polymer composition F compared to the comparative polymer comp. 3. The sample without diblock showed a too high viscosity for the application and a tendency to gel.

**Table 7 (Die-cutting ease test method 1)**

| Formulation based on... | PC. A | PC. B | PC. C | PC. E | Comp. PC. 2 | Comp. PC. 4 |
|---|---|---|---|---|---|---|
| Blade angle | | | | | | |
| 60° MB | 0 | 0 | 0 | 0 | 0 | 60 |
| 60° LE | 0 | 0 | 0 | 0 | 80 | 10 |
| 75° MB | 0 | 0 | 0 | 10 | 0 | 120 |
| 75° LE | 0 | 0 | 0 | 0 | 110 | 75 |
| 110°MB | 0 | 0 | 0 | 40 | | Too many defects |
| 110° LE | 0 | 2 | 0 | 0 | All labels were entrained with the matrix | Too many defects |

From these results, we can see that polymer compositions A, B, C and E show excellent results and outstandingly superior to comparative polymer comp. 4. Polymer composition C, with 37% diblocks, seems to outperform polymer composition E with 65% diblocks.

**Table 8 (Test method 2)**

| Formulation based on | PC. B | Comparative PC. 3 |
|---|---|---|
| Speed without problem | | |
| 60° blade angle | All speeds ok | Matrix break at all speeds |
| 75° | All speeds ok | Matrix break at all speeds |
| 110° | All speeds ok | < 50 m/min |

The die-cutting behaviour of polymer composition B greatly outperforms that of the composition based on SIS/SB (currently the bench mark in the market), which illustrates the superior properties of Polymer composition B.

### Technical field

The present invention concerns a label stock with improved die-cutting performance. More in particular, the present invention concerns labels comprising a laminate of face stock, pressure sensitive layer and release liner, wherein the pressure sensitive layer is an adhesive comprising a tackified styrenic block copolymer.

## Claims

1. A label stock comprising a laminate of face stock, a pressure sensitive adhesive layer and a release liner, wherein the pressure sensitive adhesive layer comprises:
(a) at least one block copolymer, comprising at least two terminal blocks of poly(vinyl aromatic compound) and at least one midblock of a randomly copolymerized mixture of isoprene (T) and butadiene (B) used in a weight ratio of (I):(B) of 35:65 to 80:20, and having a poly(vinyl aromatic compound) content in the range of from 10 to 35wt% and an apparent molecular weight in the range of from 180,000 to 400,000 g/mol;
(b) at least one diblock copolymer, comprising one block of poly(vinyl aromatic compound) and one block of polymerized butadiene, isoprene or mixtures thereof, wherein the weight ratio of (I):(B) is in the range of from 10:90 to 80:20,
(c) a tackifying resin; and
(d) optional ly a plasticizer,
wherein component (a) is a thermoplastic elastomer, component (b) comprises from 30 to 85% by weight of the mixture of components (a) and (b), and wherein on 100 parts by weight of the mixture of components (a) and (b), from 20 to 450 parts by weight component (c) is used.

2. A label stock according to claim 1, wherein component (a) is mainly a block copolymer, having a structure according to the formulae
S-(I/B)-S (I)
or
[S-(I/B)]ₙX (2),
wherein S represents a poly(vinyl aromatic compound) block, (I/B) represents a block of a randomly copolymerized mixture of isoprene and butadiene, wherein X represents the residue of a coupling agent, wherein n is an integer equal to or greater than 2, wherein the (I):(B) weight ratio is in the range of from 40:60 to 80:20 and wherein component (b) comprises from 35 to 85% by weight of components (a) and (b).

3. A label stock according to claims 1 and 2, wherein the weight ratio of (I):(B) in component (a) is in the range of from 45:55 to 80:20.

4. A label stock according to claims 1-3, wherein the poly(vinyl aromatic compound) content in component (a) is in the range from 13 to 30 wt%.

5. A label stock according to claims 2-4, wherein the weight ratio of (I):(B) in both the triblock copolymer S-(I/B)-S (a) and the diblock component S-(I/B) (b) is in the range of from 70:30 to 80:20.

6. A label stock according to claim 1, wherein the weight proportion of component (c) is in the range of from 100 to 200 parts by weight per 100 parts by weight of component (a) and (b).

7. A label stock according to claim 1, wherein the weight proportion of component (d) is in the range of from 20 to 50 parts by weight per 100 parts by weight of component (a) and (b).

8. A label stock according to claim 1, wherein the component (c) tackifying resin has an average aromaticity (in relative percentage of aromatic protons as determined by proton NMR) in the range of 3 to 14%.

## Patentansprüche

1. Etikettenmaterial, umfassend ein Laminat aus einem Deckmaterial, eine druckempfindliche haftfähige Schicht und ein Auslöseträgermaterial, worin die druckempfindliche haftfähige Schicht umfasst:
(a) mindestens ein Blockcopolymer, umfassend mindestens zwei terminale Blocks aus einer Poly(vinyl-aromatischen Verbindung) und mindestens einen Mittelblock aus einer beliebig polymerisierten Mischung aus Isopren (I) und Butadien (B), die in einem Gewichtsverhältnis von (I):(B) von 35:65 bis 80:20 verwendet werden, und das einen Gehalt an der Poly(vinylaromatischen-Verbindung) in dem Bereich von 10 bis 35 Gew.-% und ein apparentes Molekulargewicht in dem Bereich von 180.000 bis 400.000 g/mol hat;
(b) mindestens ein Diblockcopolymer, umfassend einen Block aus einer Poly(vinyl-aromatischen-Verbindung) und einen Block aus einem polymerisierten Butadien, Isopren oder Mischungen davon, worin das Gewichtsverhältnis von (I):(B) in dem Bereich von 10:90 bis 80:20 ist;
(c) ein klebrig machendes Harz; und
(d) optional einen Weichmacher,
worin der Bestandteil (a) ein thermoplastisches Elastomer ist, der Bestandteil (b) 30 bis 85 Gew.-% der Mischung aus den Bestandteilen (a) und (b) umfasst und worin auf 100 Gewichtsanteile von der Mischung aus den Bestandteilen (a) und (b) 20 bis 450 Gewichtsbestandteile des Bestandteils (c) verwendet werden.

2. Etikettenmaterial nach Anspruch 1, worin der Bestandteil (a) im Wesentlichen ein Blockcopolymer ist, das eine Struktur entsprechend den Formeln
S-(I/B)-S (1)
oder
[S-(IB)]ₙX (2)
hat,
worin S einen Block der Poly(vinyl-aromatischen-Verbindung) darstellt, (I/B) einen Block aus einer beliebig copolymerisierten Mischung aus Isopren und Butadien darstellt, worin X den Rest eines Kopplungsmittels darstellt, worin n eine ganze Zahl gleich oder größer als 2 ist, worin das Gewichtsverhältnis (I):(B) in dem Bereich von 40:60 bis 80:20 ist und worin der Bestandteil (b) 35 bis 85 Gew.-% der Bestandteile (a) und (b) umfasst.

3. Etikettenmaterial nach den Ansprüchen 1 und 2, worin das Gewichtsverhältnis von (I):(B) in dem Bestandteil (a) in dem Bereich von 45:55 bis 80:20 ist.

4. Etikettenmaterial nach einem der Ansprüche 1 - 3, worin der Gehalt der Poly(vinyl-aromatischen-Verbindung) in dem Bestandteil (a) in dem Bereich von 13 bis 30 Gew.-% ist.

5. Etikettenmaterial nach einem der Ansprüche 2 - 4, worin das Gewichtsverhältnis von (I):(B) in sowohl dem Triblockcoplymer S-(I/B)-S (a) als auch dem Diblockbestandteil S-(I/B) (b) in dem Bereich von 70:30 bis 80:20 ist.

6. Etikettenmaterial nach Anspruch 1, worin der Gewichtsanteil des Bestandteils (c) in dem Bereich von 100 bis 200 Gewichtsanteilen pro 100 Gewichtsanteilen des Bestandteils (a) und (b) ist.

7. Etikettenmaterial nach Anspruch 1, worin der Gewichtsanteil des Bestandteils (d) in dem Bereich von 20 bis 50 Gewichtsanteilen pro 100 Gewichtsanteilen des Bestandteils (a) und (b) ist.

8. Etikettenmaterial nach Anspruch 1, worin der Bestandteil (c), das klebrig machende Harz, eine durchschnittliche Aromatizität (relativer Prozentsatz an aromatischen Protonen, wie durch eine Protonen-NMR bestimmt wurde) in dem Bereich von 3 bis 14 % hat.

## Revendications

1. Structure d'étiquette comprenant un stratifié constitué d'un frontal, d'une couche adhésive de contact et d'un support anti-adhésif, dans laquelle la couche adhésive de contact comprend :
(a) au moins un copolymère à blocs comprenant au moins deux blocs terminaux de poly(composé vinylaromatique) et au moins un bloc central d'un mélange copolymérisé par polymérisation aléatoire d'isoprène (I) et de butadiène (B) utilisés selon un rapport en poids (I):(B) de 35:65 à 80:20 et ayant une teneur en le poly(composé vinylaromatique) comprise dans la plage de 10 à 35 % en poids et une masse moléculaire apparente comprise dans la plage de 180 000 à 400 000 g/mole ;
(b) au moins un copolymère dibloc comprenant un bloc de poly(composé vinylaromatique) et un bloc de butadiène ou d'isoprène polymérisé ou de mélanges de ceux-ci, le rapport en poids (I):(B) étant compris dans la plage de 10:90 à 80:20,
(c) une résine de pégosité (ou résine tackifiante) ; et
(d) en option un plastifiant,
où le composant (a) est un élastomère thermoplastique, le composé (b) représente 30 à 85 % en poids du mélange des composants (a) et (b), et, pour 100 parties en poids du mélange de composants (a) et (b), on utilise de 20 à 450 parties en poids du composant (c).

2. Structure d'étiquette selon la revendication 1, dans laquelle le composant (a) est essentiellement un copolymère à blocs ayant une structure correspondant aux formules
S-(I/B)-S (1)
ou
[S-(I/B)]ₙS (2),
où S représente un bloc poly(composé vinylaromatique), (I/B) représente un bloc constitué d'un mélange copolymérisé par polymérisation aléatoire d'isoprène et de butadiène, où X représente le résidu d'un agent de couplage, n est un entier supérieur ou égal à 2, le rapport en poids (I):(B) est compris dans la plage de 40:60 à 80:20, et le composant (b) représente 35 à 85 % en poids des composants (a) et (b).

3. Structure d'étiquette selon les revendications 1 et 2, dans lequel le rapport en poids (I):(B) dans le composant (a) et compris dans la plage de 45:55 à 80:20.

4. Structure d'étiquette selon les revendications 1 à 3, dans laquelle la teneur du composant (a) en le poly(composé vinylaromatique) est comprise dans la plage de 13 à 30 % en poids.

5. Structure d'étiquette selon les revendications 2 à 4, dans lequel le rapport en poids (I):(B), tant dans le copolymère tribloc S-(I/B)-S (a) ou dans le composant dibloc S-(I/B) (b) est compris dans la plage de 70:30 à 80:20.

6. Structure d'étiquette selon la revendication 1, dans lequel la proportion pondérale du composant (c) est comprise dans la plage de 100 à 200 parties en poids pour 100 parties en poids des composants (a) et (b).

7. Structure d'étiquette selon la revendication 1, dans laquelle la proportion pondérale du composant (d) est comprise dans la plage de 20 à 50 parties en poids pour 100 parties en poids des composants (a) et (b).

8. Structure d'étiquette selon la revendication 1, dans laquelle la résine de pégosité (ou résine tackifiante) du composant (c) a une aromaticité moyenne (en pourcentage relatif des protons aromatiques, tel que déterminé par RMN de proton) comprise dans la plage de 3 à 14 %.
